# EUROPÄISCHE PATENTSCHRIFT

(11) **EP 1 569 890 B1**
(45) Veröffentlichungstag und Bekanntmachung des Hinweises auf die Patenterteilung: **23.03.2011**
(21) Anmeldenummer: 03785708.3
(22) Anmeldetag: 02.12.2003
(51) Int. Cl.: C07C 67/08, C07C 69/24

(54) **VERFAHREN ZUR HERSTELLUNG VON LINEAREN BZW. VERZWEIGTEN FETTSÄUREESTERN DURCH HETEROGEN KATALYSIERTE REAKTIVE REKTIFIKATION MIT VORREAKTOR**
METHOD FOR PRODUCING LINEAR OR BRANCHED FATTY ACID ESTERS BY MEANS OF HETEROGENEOUSLY CATALYSED REACTIVE RECTIFICATION WITH AN UPSTREAM REACTOR
PROCEDE DE FABRICATION D'ESTERS D'ACIDES GRAS LINEAIRES OU RAMIFIES PAR RECTIFICATION REACTIVE A CATALYSE HETEROGENE A L'AIDE D'UN REACTEUR AMONT

(30) Priorität: 10.12.2002 DE 10257525
(43) Veröffentlichungstag der Anmeldung: 07.09.2005
(73) Patentinhaber: Cognis IP Management GmbH, 40589 Düsseldorf (DE)
(72) Erfinder: GUTSCHE, Bernhard, 40724 Hilden (DE); TOPPHOFF, Magnus, 40625 Düsseldorf (DE); RÖSSLER, Harald, 40593 Düsseldorf (DE); REUTER, Erich, Greer, SC, 29651 (US)
(74) Vertreter: Gittinger, Andreas
(86) Internationale Anmeldenummer: PCT/EP2003/013563
(87) Internationale Veröffentlichungsnummer: WO 2004/052824

(56) Entgegenhaltungen:
- EP-A- 0 141 975
- EP-A- 0 474 996
- DATABASE WPI Section Ch, Week 199339 Derwent Publications Ltd., London, GB; Class A60, AN 1993-309881 XP002278667 & SU 671 223 A (DYSHLOVOI V I), 23. Oktober 1992 (1992-10-23)

## Beschreibung

### Gebiet der Erfindung

Vorgeschlagen wird ein neues Verfahren zur kontinuierlichen Veresterung von C₁-C₂₂-(Fett)säuren mit C₁-C₁₀-Monoalkanolen, C₂-C₅- Di- bzw. Trialkanolen, oder Mischungen hiervon, in flüssiger Phase im Gegenstromverfahren, in Gegenwart von heterogenen Katalysatoren im Vorreaktor und in der Reaktionskolonne.

### Stand der Technik

Verfahren zur kontinuierlichen Veresterung von Fettsäuren sind bekannt, vgl. H. Stage, Chemiker-Ztg./Chem.Apparatur 87, Nr. 18, 661 - 666 (1963**)**. Die Veröffentlichung beschreibt die Veresterung von Fettsäuren mit Methanol und n-Butanol in bei Normaldruck betriebenen mehrteiligen Reaktionskolonnen mit Dampfprallböden. Üblicherweise erfolgt die technische kontinuierliche Fettsäureveresterung jedoch mehrstufig in Bodenkolonnen bei Drücken von 5 - 30 bar, vgl. DE 2503195 C.

In der EP 0 334 154 B1 (Henkel) wird ein Verfahren zur kontinuierlichen, homogen katalysierten Veresterung von Fettsäuren mit Alkanolen im Gegenstrom in flüssiger Phase in einer Reaktionskolonne beschrieben, wobei Katalysatoren und Fettsäuren auf den obersten und Alkanole an den untersten Boden gegeben und bei einem Kopfdruck der Reaktionskolonne von 200 - 900 hPa umgesetzt werden. Durch dieses Verfahren sollen unerwünschte Nebenreaktionen, wie z.B. die Dehydratisierung von verzweigten Monoalkanolen vermieden werden. Bei diesem homogen katalysierten Verfahren muss am Ende der Reaktion zusätzlich der homogene Katalysator entfernt werden. Dadurch ergeben sich höhere Verluste an Katalysatormenge, jedoch auch an Produkt. Aus der WO 90/11114 (Henkel) ist ein diskontinuierliches Verfahren zum Führen einer heterogen katalysierten Reaktion bekannt.

Allgemein wird bei diskontinuierlichen, heterogen katalysierten Verfahren der feste Katalysator bei der direkten Zugabe in den Reaktor durch Rührorgane zerkleinert und muß nach der Reaktion abfiltriert werden. In der WO 90/11114 wird nun ein Verfahren beschrieben, bei dem die Edukte in einem Reaktor vorgemischt und anschließend mittels Pumpen in einen außenliegenden Katalysatorbehälter eingebracht werden. Das Reaktionsgemisch strömt anschließend durch einen Dünnschicht- oder Fallfilmverdampfer. Durch die stetige Kontrolle z.B. der Säurezahl wird der Reaktionsfortschritt ermittelt. Bei diesem Verfahren ist nachteilig zu bewerten, dass es sich um ein diskontinuierliches Verfahren handelt, Als Nachteile der diskontinuierlichen Fahrweise sind nach E. Fitzer, Technische Chemie, 4 Auflage, (1995**)** auftretende Totzeiten beim Füllen, Entleeren, Aufheizen und Abkühlen, höhere Energiekosten und größerer Personalaufwand zu nennen.

Aus der EP 0474996 A1 (CW Hüls) ist ein Verfahren zur Herstellung von Estern aus Alkoholen und Säuren durch flüssigphasige Gleichgewichtsreaktionen an Ionenaustauschern bekannt. Der Reaktion wird in einem Vorreaktor und einer Rektifikationskolonne mit zusätzlichen, außenliegenden Reaktoren durchgeführt. Wegen der hohen thermischen Belastung des lonenaustauschers in einer Reaktionsdestillationskolonne und der schwierigen destillativen Trennung in Gegenwart von Ionenaustauschern muß die Veresterung und Rektifikation räumlich getrennt durchgeführt werden. Durch das erfindungsgemäße Verfahren werden diese Nachteile jedoch zuverlässig überwunden.

Gegenstand der SU 671223 ist ein Verfahren zur Veresterung von Carbonsäuren, wobei die Alkoholkomponente auf Methanol beschränkt ist. Das Verfahren ist durch zwei Veresterungsschritte gekennzeichnet, wobei zunächst die Carbonsäuren und eine erste Menge Methanol bis zu einem unbekannten Veresterungsgrad umgesetzt werden. Zwischen den beiden Veresterungsschritten wird das Zwischenprodukt einer Rektifikation unterworfen, wobei Wasser und Methanol abgetrennt werden. Das Intermediat wird dann mit frischem Methanol versetzt und in einem zweiten Reaktor weiter verestert. Aus der EP 0141975 A2 (CW Hüls) ist ein Verfahren zur Herstellung von Essigsäureestern bekannt, bei dem ebenfalls zwischen Vor- und Hauptveresterung unterschieden wird.

Aufgabe war es ein wirtschaftliches Verfahren zur Veresterung von (Fett)säuren mit Alkanolen zu entwickeln, wobei das Verfahren ein kontinuierliches, heterogen katalysiertes Verfahren sein sollte, frei von den bereits geschilderten Nachteilen. Weiterhin sollten die Farbe und der Geruch der Produkte, durch eine geringere thermische Belastung während der Herstellung verbessert werden.

Insbesondere war es wichtig ein verbessertes Verfahren zur Veresterung von Alkoholen und Säuren in Gegenwart von thermisch wenig belastbaren Ionenaustauschern zu entwickeln, bei dem es nicht nötig ist, apparativ die Trennung von Ionenaustauscher und Rektifikationskolonne durchzuführen. Beim erfindungsgemäßen Verfahren befindet sich der Katalysator sowohl im Vorreaktor als auch in der Rektifikationskolonne. Eine räumliche Trennung ist nicht nötig. Diese Aufgaben wurden durch das erfindungsgemäße Verfahren gelöst.

### Beschreibung der Erfindung

Gegenstand der Erfindung ist Verfahren zur kontinuierlichen Veresterung von C₁-C₂₂-(Fett)säuren mit C₁-C₁₀-Monoalkanolen, C₂-C₅-Di- bzw. Trialkanolen, oder Mischungen hiervon, in flüssiger Phase im Gegenstromverfahren, in Gegenwart von heterogenen Katalysatoren im Vorreaktor (1) und in der Reaktionskolonne (3), dadurch gekennzeichnet, dass
(i) ein Festbett-Vorreaktor (1) und eine Abtrenneinheit ausgewählt aus der Gruppe die gebildet wird von Flashbehältern und Phasenabscheidern (2) der Reaktionskolonne (3) zur Verringerung der Viskosität des Reaktionsgemisches vorgeschaltet wird und
(ii) über die Abtrenneinheit (2), zur Verschiebung des Reaktionsgleichgewichts vor der Reaktionskolonne (3) das Reaktionswasser aus dem System entfernt wird.

Beim erfindungsgemäßen Verfahren wird das Reaktionsgemisch aus (Fett)säuren und Alkanolen durch einen Festbettreaktor (Vorreaktor) geleitet. Hier findet eine Teilumsetzung zu den entsprechenden Estern statt. Dieses bereits teilumgesetzte, wesentlich niedrigviskosere Reaktionsgemisch wird, nach Abtrennung des bei der Reaktion entstehenden Wassers durch eine Abtrenneinheit, in die mit Katalysator beschickte Reaktionskolonne eingeleitet. Durch den Einsatz eines Vorreaktors kann somit die Viskosität des Eduktstromes deutlich abgesenkt werden, wodurch eine gleichmäßige Durchströmung des Festbettes in der Kolonne erreicht wird. Das vorzeitige Abscheiden des Reaktionswassers vor der weiteren Veresterungsreaktion in der Reaktionskolonne verschiebt das Reaktionsgleichgewicht zugunsten der entstehenden Ester. Es ergeben sich verbesserte Produkteigenschaften, sowie eine Erhöhung der Ausbeuten. So können im beschriebenen Prozess Umsätze bis zu 99.9% bezogen auf die eingesetzte schwerer flüchtige Komponente erreicht werden. Des Weiteren werden durch das erfindungsgemäße Verfahren Folge- bzw. Nebenreaktionen unterdrückt. Neben dem Vorteil der einfachen Regenerierung des Katalysators im Vorreaktor, wirkt er gleichzeitig auch als "Scavenger" zum Abfangen möglicher Katalysatorgifte, wodurch die Standzeiten des Katalysators in der Reaktionskolonne deutlich verlängert werden und so geringere Produktionskosten realisiert werden können.

### Anlage

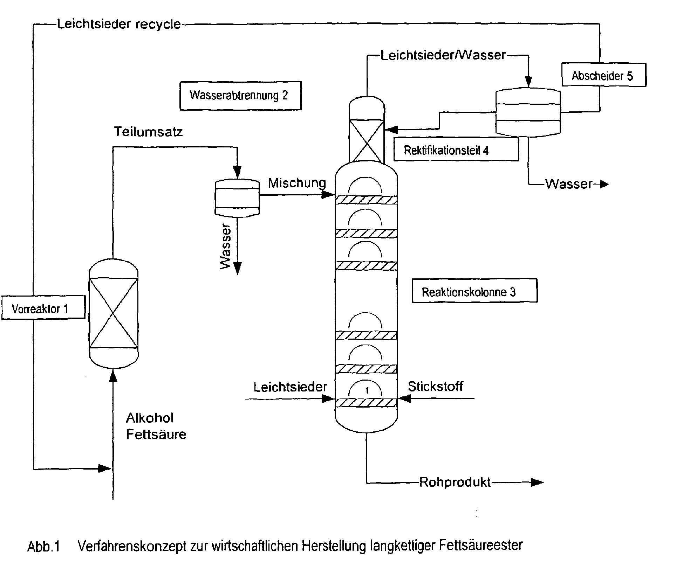

Das Verfahren der Erfindung wird im Folgenden anhand einer bevorzugten Anlage erläutert, wobei auf die Zeichnung (Abb. 1) Bezug genommen wird. Dies soll jedoch den Gegenstand der Erfindung nicht beschränken. Zentrales Element einer solchen Anlage ist der Vorreaktor (1), der die Wasserabtrennung (Abtrenneinheit) (2) und eine Vielzahl von Glockenböden aufweisende Reaktionskolonne (3) mit aufgesetztem Rektifikationsteil (4). Sowohl der Vorreaktor als auch in die Reaktionskolonne sind mit Katalysator befüllt. Säuren und Alkanole werden durch den mit Katalysator gefüllten Vorreaktor geleitet. Hier findet eine Teilumsetzung zum Ester statt. Das vorveresterte Produkt wird flüssig, und bereits in seiner Viskosität erniedrigt, durch eine Abtrenneinheit (2) geleitet, die sich zwischen Vorreaktor und Reaktionskolonne befindet. Dabei wird das entstehende Reaktionswasser aus dem teilumgesetzten Reaktionsgemisch entfernt. Dadurch wird das Gleichgewicht der Veresterungsreaktion in Richtung Produkte verschoben, was zu einer erhöhten Ausbeute an Estern führt.

Die Abtrennung des Reaktionswassers wird, je nach eingesetztem Alkanol, mittels Flashverfahren oder mit einem Phasenabscheider durchgeführt. Dabei wird je nach eingesetztem Alkohol das Wasser unterschiedlich abgetrennt. So wird beim Einsatz der kurzkettigen Alkohole Methanol bzw. Ethanol das Wasser mit Hilfe eines Flashbehälters zusammen mit einem Teil des Alkohols aus dem Prozeßstrom entfernt. Auf dieselbe Weise wird beim Einsatz kurzkettiger Carbonsäuren (z.B. Essigsäure bei der Triacetinherstellung) das entstehende Wasser zusammen mit einem Teil der Essigsäure aus dem Strom ausgeschleust. Unter Verwendung anderer Alkohole, wie Butanol bzw. 2-Ethylhexanol kann ein Phasenabscheider zur Abtrennung des Wassers verwendet werden. Weiterhin können geeignete Membranverfahren zur Abtrennung genutzt werden.

Die teilumgesetzte Reaktionsmischung wird sodann in die Reaktionskolonne (3) eingespeist. Der heterogene Katalysator ist direkt auf den Kolonnenböden aufgebracht. Die Kolonne (3) wird im Gegenstromverfahren betrieben. Allgemein werden hierbei die leichter siedenden Komponenten, z.B. Alkanole im Sumpf der Kolonne eingespeist, die schwerer siedenden Komponenten, wie das teilumgesetzte Reaktionsprodukt, am obersten Boden der Reaktivrektifikationskolonne (3). Im Falle der Veresterung von Glycerin mit Essigsäure wird jedoch die Säure als leichter siedende Komponente im Sumpf der Kolonne eingespeist.

Die weitere Veresterungsreaktion findet in der Kolonne statt. Das entstehende Reaktionsprodukt wird im Sumpf der Kolonne abgezogen. Eine weitere Aufarbeitung des Rohproduktes erfolgt durch Destillation und gegebenenfalls durch Desodorierung. Die durch die Destillation abgetrennten Komponenten können dem Prozeß durch Einleitung in den Vorreaktor (1) und/oder durch Einspeisung in den Sumpf der Kolonne (3) wieder zugeführt werden.

Im aufgesetzten Rektifikationsteil (4) der Reaktionskolonne wird ein Gemisch aus Leichtsieder (Alkanol bzw. Säure) und Wasser abdestilliert. Je nach verwendetem Leichtsieder sieht die weitere Aufarbeitung des Gemisches über die Abtrenneinheit (5) unterschiedlich aus. So wird beim Einsatz der kurzkettigen Alkohole Methanol bzw. Ethanol die Trennung von Wasser mit Hilfe einer zusätzlichen Kolonne vorgenommen. Unter Verwendung anderer Alkohole, wie Butanol bzw. 2-Ethylhexanol kann ein Phasenabscheider zur Abtrennung des Wassers verwendet werden. Als weitere Möglichkeit sind auch Membranverfahren zur Überwindung z.B. azeotroper Punkte im System einsetzbar.

Der abgetrennte Leichtsieder wird teilweise als Rückfluss wieder in die Kolonne (3) bzw. als Feed in den Vorreaktor (1) eingespeist. Das erfindungsgemäße Verfahren zeichnet sich weiterhin dadurch aus, dass Stickstoff als zusätzliches Strippmittel (Schleppmittel) zur Entfernung des Reaktionswassers verwendet wird. Der Stickstoff wird am untersten Boden in die Kolonne eingespeist. Durch den Einsatz des Stickstoffs wird außerdem die Dampfbelastung im unteren Teil der Kolonne erhöht, wodurch ein "Durchregnen" der flüssigen Phase verhindert wird. Weiterhin wird durch den Einsatz des Stickstoffs ein günstigeres Einsatzverhältnis (Fett)säure zu Alkohol erreicht. Bei festgelegter, benötigter Dampfbelastung in der Kolonne kann unter Verwendung von Stickstoff eine geringere Alkoholmenge eingesetzt werden, als bei der konventionellen Fahrweise. Dies führt zu einer wirtschaftlich vorteilhafteren Produktion. Gleichzeitig bewirkt die Einleitung von Stickstoff durch die Reaktionsko-Ionne eine Desodorierung der Reaktionsmischung. Es können so Produkte erhalten werden, die gegebenenfalls bereits ohne zusätzliche Desodorierung einen sensorisch einwandfreien Geruch aufweisen. Dies ist besonders vorteilhaft für den Einsatz dieser Produkte in Kosmetika.

In einer bevorzugten Ausführungsform wird deshalb ein Verfahren beansprucht, welches dadurch gekennzeichnet ist, dass Stickstoff am untersten Boden der Reaktionskolonne (3) zugespeist wird um die Dampfbelastung im unteren Teil der Reaktionskolonne zu erhöhen. Insbesondere bevorzugt ist weiterhin ein Verfahren, dadurch gekennzeichnet, dass Stickstoff als Strippmittel am untersten Boden der Reaktionskolonne (3) zugespeist wird zur zusätzlichen Entfernung des Reaktionswassers. Weiterhin bevorzugt ist ein Verfahren, welches dadurch gekennzeichnet ist, dass Stickstoff zur Desodorierung am untersten Boden der Reaktionskolonne (3) zugespeist wird.

### Vorreaktor

In einer bevorzugten Ausführungsform ist der Vorreaktor (1) ein Festbettreaktor. Im Festbettreaktor wird das Katalysatormaterial durch geeignete Elemente, z.B. durch Spaltsiebe, zurückgehalten.

### Reaktionskolonne und Rektifikationsteil

Als für das Verfahren geeignete Reaktionskolonnen (3) sind allgemein sämtliche üblichen Bodenkolonnen wie Siebbodenkolonnen zu nennen, insbesondere jedoch Glockenbodenkolonnen mit hohen Flüssigkeitsständen. Typische Vertreter dieser Kolonnen sind in der EP - B-0033929 **und** DE-A-3146142 beschrieben. Die Verwendung dieser Kolonnengeneration ist vorteilhaft, da ein geringerer Alkoholüberschuss als in konventionellen Kolonnen realisiert werden muß, um einen vollständigen Umsatz zu erreichen. Der Katalysator ist direkt auf den Kolonnenböden in der Reaktionskolonne (3) aufgebracht.

### Temperatur und Druck

Die Reaktion findet im Festbettreaktor bei Temperaturen zwischen 50 und 150, vorzugsweise 80 bis 120 °C und Drücken zwischen 1 und 10 bar, vorzugsweise 1 bis 5 bar statt. Die Veresterung in der reaktiven Rektifikationskolonne findet bei Temperaturen zwischen 50 und 200, vorzugsweise 80 bis 150 °C und Drücken zwischen 0,1 und 10 bar, vorzugsweise 0.1 bis 5 bar statt. In einer bevorzugten Ausführungsform wird deshalb ein Verfahren beansprucht, welches dadurch gekennzeichnet ist, dass die Veresterung bei Temperaturen zwischen 50 und 200 °C, vorzugsweise 80 bis 150°C durchgeführt wird.

### Alkanole

Geeignete Alkanole sind lineare oder verzweigte Monoalkanole, Di- bzw. Trialkanole oder Mischungen hiervon. In einer bevorzugten Ausführungsform werden lineare oder verzweigte Monoalkohole mit 1 bis 10 vorzugsweise 1 bis 8 Kohlenstoffatomen eingesetzt. Es handelt sich hierbei beispielsweise um Methanol, Ethanol, Propanol, Butanol, Pentanol und Hexanol sowie deren Isomere. Insbesondere bevorzugt werden die (Fett)säuren mit iso-Propanol oder 2-Ethylhexanol verestert. Als lineare oder verzweigte Di- bzw. Trialkanole mit 2 bis 5 Kohlenstoffatomen können beispielsweise Glycerin, Ethandiol, 1,2-Propandiol, 1,3-Propandiol, Butandiol und Pentandiol, deren Isomere, sowie deren Halbester eingesetzt werden. In einer vorteilhaften Ausführungsform werden die (Fett)säuren deshalb mit C₂ bis C₅ Di- bzw. Trialkanolen, vorzugsweise C₂ bis C₃ Di- bzw. Trialkanolen, insbesondere mit Glycerin verestert.

### (Fett)säuren

Als Ausgangsprodukte zur Herstellung der Ester eignen sich (Fett)säuren mit insgesamt 1 bis 22 Kohlenstoffatomen. Unter den Begriff (Fett)säuren sind sowohl ein- und mehrwertige Carbonsäuren als auch aliphatische Fettsäuren zu verstehen. Geeignete Carbonsäuren sind Ameisensäure, Essigsäure, sowie Adipinsäure, Dodecandisäure, Citronensäure, Isophthalsäure. Unter aliphatischen Fettsäuren sind aliphatische Carbonsäuren der Formel (I) zu verstehen,

R¹CO-OH (I)

in der R¹CO für einen aliphatischen, linearen oder verzweigten Acylrest mit 6 bis 22 Kohlenstoffatomen und 0 und/oder 1, 2 oder 3 Doppelbindungen steht. Typische Beispiele sind Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder der Dimerisierung von ungesättigten Fettsäuren anfallen. Bevorzugt sind technische Fettsäuren mit 12 bis 18 Kohlenstoffatomen, wie beispielsweise Kokos-, Palm-, Palmkern- oder Talgfettsäure. Weiterhin bevorzugt ist der Einsatz von Vorlauffettsäuren mit 6 - 12 Kohlenstoffatomen, der insbesondere bei der Aufarbeitung von Fettsäuregemischen natürlichen Ursprungs in großen Mengen anfällt.

Gemäß einer bevorzugten Ausführungsform werden C₆ bis C₂₂ (Fett)säuren, vorzugsweise C₈ bis C₁₈ (Fett)säuren, insbesondere C₁₀ bis C₁₆ (Fett)säuren eingesetzt. Gemäß einer weiteren vorteilhaften Ausführungsform wird ein Verfahren beansprucht, bei dem C₁ bis C₅ Carbonsäuren mit C₂ bis C₃ Di- bzw. Trialkanolen, insbesondere Essigsäure mit Glycerin verestert wird.

### Katalysator

Beim erfindungsgemäßen Verfahren werden Katalysatoren eingesetzt, die ausgewählt sind aus der Gruppe, die gebildet wird von basischen oder sauren Anionen- oder Kationenaustauscher auf organischer oder anorganischer Basis oder sauer eingestellten Tonerden, Zeolithen oder speziell aufgearbeiteten Bleicherden, sowie Katalysatoren auf Basis von Übergangsmetallen. Besonders bevorzugt ist der Einsatz von stark sauren Kationenaustauscher, Katalysatoren auf der Basis von Übergangsmetalloxiden bzw. organofunktionellen Polysiloxanen. In einer besonderen Ausführungsform ist der Einsatz von sauren Kationenaustauschern als Katalysator bevorzugt. Beispielhaft wird hier auf den Katalysator der Firma Rohm & Haas Amberlyst 17 (XE-386) verwiesen. Vorzugsweise setzt man die schwerer siedende Komponente (z.B. (Fett)säuren) zur leichter siedenden Komponente (z.B. Monoalkanole) in molaren Verhältnissen - berechnet für die Umsetzung einer Carboxylgruppe mit einer Hydroxylgruppe - von maximal 1:2, vorzugsweise von 1:1,5, insbesondere 1:1 ein. D.h. die leichter siedende Komponente soll maximal mit zweifachem Überschuss vorliegen.

Werden mehrwertige (Fett)säuren, bzw. Di- oder Trialkanole eingesetzt, muß das Molverhältnis unter Berücksichtigung der gewünschten Produkte (Partial-und/oder Vollester) entsprechend multipliziert und angepasst werden. Wiederum soll gelten, dass die leichter siedende Komponente maximal im zweifachen Überschuss vorliegt. Beispielhaft sei hier aufgezeigt, dass für die Umsetzung von Glycerin (schwerer siedende Komponente) mit Essigsäure (leichter siedende Komponente) diese maximal im Verhältnis 1:6 (d.h. zweifacher Überschuss der Essigsäure), vorzugsweise 1:4,5, insbesondere 1:3 eingesetzt werden.

### Beispiele

### Beispiel 1

Als Katalysator für die Reaktion wird Amberlyst 17 (XE-386) der Firma Rohm & Haas verwendet. Der Katalysator wird in einem beheizten Glasdoppelmantel (Vmax = 200ml; D= 20 mm) als Festbettreaktor und in der Reaktionskolonne (27 Glockenböden, D = 50 mm, 1 Glocke pro Boden) vorgelegt. Die folgende Veresterung von Essigsäure mit Glycerin wird bei 85 °C durchgeführt. Das Glycerin wird zusammen mit einem Teil der Essigsäure (ES/Gly = 4/1 [mol/mol]) in den Vorreaktor eingespeist. Der Volumenstrom beträgt 169 g/h. Nach der ersten Reaktionsstufe beträgt der Umsatz 56 %. Das vorveresterte Produkt wird flüssig am obersten Boden der Reaktionskolonne eingespeist. Im Gegenstrom wird die Essigsäure überhitzt und dampfförmig mit einem Volumenstrom von 268 g/h eingesetzt. Essigsäure/Wasser wird am Kopf der Kolonne und das Reaktionsprodukt im Sumpf der Kolonne abgezogen. Der Kopfdruck innerhalb der Kolonne beträgt 200 mbar. Der Umsatz bezogen auf Glycerin beträgt 88.7 % im Sumpf. Um auf den gewünschten Umsatz >98 % zu kommen wird die Kolonne ein zweites Mal durchlaufen, wobei ein nahezu vollständiger Umsatz (99.9%) bezogen auf Glycerin erreicht wird. Die Betriebsbedingungen wurden analog dem ersten Durchlauf gewählt. Die Zusammensetzung der Produktmuster nach Durchlauf eins und zwei sind in Tabelle 1 wiedergegeben. Zur Aufarbeitung des Reaktionsproduktes erfolgt eine Nachreaktion mit Essigsäureanhydrid, eine Essigsäureabtrennung und eine Triacetindestillation.

### Tabelle 1

Zusammensetzung der Produktmuster

| Massenanteile [Gew.-%] | | | | | | |
|---|---|---|---|---|---|---|
| Durchlauf | Essigsäure | Glycerin | Wasser | Monoacetin | Diacetin | Triacetin |
| 1 | 52,0 | 0,1 | 0,6 | 0,3 | 13,7 | 33,4 |
| 2 | 50,6 | - | <0,1 | - | 0,7 | 48,7 |

### Beispiel 2

Die Vorgehensweise in Beispiel 1 wird wiederholt und als Katalysator für die Reaktion wird Amberlyst 17 (XE-386) der Firma Rohm & Haas verwendet. Die folgende Veresterung von Essigsäure mit Glycerin wird bei 85 °C durchgeführt. Das Glycerin wird zusammen mit einem Teil der Essigsäure (ES/Gly = 1/1 [mol/mol]) in den Vorreaktor eingespeist. Der Volumenstrom beträgt 174 g/h. Nach der ersten Reaktionsstufe beträgt der Umsatz 23.5 %. Das vorveresterte Produkt wird flüssig am obersten Boden der Reaktionskolonne eingespeist. Im Gegenstrom wird die Essigsäure dampfförmig mit einem Volumenstrom von 262 g/h eingesetzt. Essigsäure/Wasser wird am Kopf der Kolonne und das Reaktionsprodukt im Sumpf der Kolonne abgezogen. Der Umsatz bezogen auf Glycerin beträgt 65 % im Sumpf. Zur Aufarbeitung des Reaktionsproduktes erfolgt eine Nachreaktion mit Essigsäureanhydrid, eine Essigsäureabtrennung und eine Triacetindestillation.

## Patentansprüche

1. Verfahren zur kontinuierlichen Veresterung von C₁-C₂₂-(Fett)säuren mit C₁-C₁₀-Monoalkanolen, C₂-C₅-Di- bzw. Trialkanolen, oder Mischungen hiervon, in flüssiger Phase im Gegenstromverfahren, in Gegenwart von heterogenen Katalysatoren im Vorreaktor (1) und in der Reaktionskolonne (3), **dadurch gekennzeichnet, dass**
(i) ein Festbett-Vorreaktor (1) und eine Abtrenneinheit ausgewählt aus der Gruppe die gebildet wird von Flashbehältern und Phasenabscheidern (2) der Reaktionskolonne (3) zur Verringerung der Viskosität des Reaktionsgemisches vorgeschaltet wird und
(ii) über die Abtrenneinheit (2), zur Verschiebung des Reaktionsgleichgewichts vor der Reaktionskolonne (3) das Reaktionswasser aus dem System entfernt wird.

2. Verfahren nach Anspruch 1, **dadurch gekennzeichnet, dass** Stickstoff am untersten Boden der Reaktionskolonne (3) zugespeist wird, um die Dampfbelastung im unteren Teil der Reaktionskolonne zu erhöhen.

3. Verfahren nach Anspruch 1 und/oder 2, **dadurch gekennzeichnet, dass** Stickstoff als Strippmittel am untersten Boden der Reaktionskolonne (3) zugespeist wird zur zusätzlichen Entfernung des Reaktionswassers.

4. Verfahren nach einem der Ansprüche 1 bis 3, **dadurch gekennzeichnet, dass** Stickstoff zur Desodorierung am untersten Boden der Reaktionskolonne (3) zugespeist wird.

5. Verfahren nach einem der Ansprüche 1 bis 4, **dadurch gekennzeichnet, dass** die Veresterung bei Temperaturen zwischen 50 und 200 °C durchgeführt wird.

6. Verfahren nach einem der Ansprüche 1 bis 5, **dadurch gekennzeichnet, dass** man die (Fett)säuren mit C₁-C₁₀-Monoalkanolen verestert.

7. Verfahren nach einem der Ansprüche 1 bis 6, **dadurch gekennzeichnet, dass** man die (Fett)säuren mit C₂-C₅-Di- bzw. Trialkanolen verestert.

8. Verfahren nach einem der Ansprüche 1 bis 7, **dadurch gekennzeichnet, dass** als (Fett)-säuren bevorzugt C₆-C₂₂-Fettsäuren eingesetzt werden.

9. Verfahren nach einem der Ansprüche 1 bis 8, **dadurch gekennzeichnet, dass** man C₁-C₅ Carbonsäuren mit C₂-C₃-Di- bzw. Trialkanolen verestert.

10. Verfahren nach einem der Ansprüche 1 bis 9, **dadurch gekennzeichnet, dass** man Veresterungskatalysatoren ausgewählt aus der Gruppe, die gebildet wird von basischen oder sauren Anionen- oder Kationenaustauscher auf organischer oder anorganischer Basis oder sauer eingestellten Tonerden, Zeolithen oder speziell aufgearbeiteten Bleicherden, sowie Katalysatoren auf Basis von Übergangsmetallen einsetzt.

11. Verfahren nach einem der Ansprüche 1 bis 10, **dadurch gekennzeichnet, dass** als Katalysatoenr saure Kationenaustauscher eingesetzt werden.

## Claims

1. A process for the continuous esterification of C₁₋₂₂ (fatty) acids with C₁₋₁₀ monoalkanols, C₂₋₅ di- or trialkanols or mixtures thereof in the liquid phase in countercurrent operation in the presence of heterogeneous catalysts in a preliminary reactor (1) and in a reaction column (3), **characterized in that**
(i) the reaction column (3) is preceded by a fixed-bed preliminary reactor (1) and a separation unit selected from the group formed by flash vessels and phase separators (2), for the purpose of reducing the viscosity of the reaction mixture and
(ii) the water of reaction is removed from the system via a separation unit (2) to displace the reaction equilibrium before the reaction column (3).

2. A process as claimed in claim 1, **characterized in that** nitrogen is fed in at the lowermost plate of the reaction column (3) in order to increase the vapor load in the lower part of the reaction column.

3. A process as claimed in claim 1 and/or 2, **characterized in that** nitrogen is fed in as stripping agent at the lowermost plate of the reaction column (3) for additionally removing the water of reaction.

4. A process as claimed in any of claims 1 to 3, **characterized in that** nitrogen is fed in at the lowermost plate of the reaction column (3) for the purpose of deodorization.

5. A process as claimed in any of claims 1 to 4, **characterized in that** the esterification is carried out at temperatures of 50 to 200°C.

6. A process as claimed in any of claims 1 to 5, **characterized in that** the (fatty) acids are esterified with C₁₋₁₀ monoalkanols.

7. A process as claimed in any of claims 1 to 6, **characterized in that** the (fatty) acids are esterified with C₂₋₅ di- or trialkanols.

8. A process as claimed in any of claims 1 to 7, **characterized in that** the (fatty) acids used are preferably C₆₋₂₂ fatty acids.

9. A process as claimed in any of claims 1 to 8, **characterized in that** C₁₋₅ carboxylic acids are esterified with C₂₋₃ di- or trialkanols.

10. A process as claimed in any of claims 1 to 9, **characterized in that** the esterification catalysts used are selected from the group consisting of organic or inorganic, basic or acidic anion or cation exchangers or acidic clays, zeolites or specially worked up bleaching earths and catalysts based on transition metals.

11. A process as claimed in any of claims 1 to 10, **characterized in that** acidic cation exchangers are used as the catalyst.

## Revendications

1. Procédé d'estérification continue d'acides (gras) en C₁-C₂₂ avec des monoalcools en C₁-C₁₀, des dialcanols ou des trialcanols en C₂-C₅, ou leurs mélanges, en phase liquide dans un procédé à contre-courant, en présence de catalyseurs hétérogènes dans le préréacteur (1) et dans la colonne de réaction (3), **caractérisé en ce que**
(i) un préréacteur à lit fixe (1) et une unité de séparation, choisie dans le groupe formé par des récipients flash et des séparateurs de phases (2) sont disposés en amont de la colonne de réaction (3) en vue de la diminution de la viscosité du mélange réactionnel et
(ii) l'eau de réaction est éliminée du système en amont de la colonne de réaction (3) via l'unité de séparation (2) en vue du déplacement de l'équilibre de réaction.

2. Procédé selon la revendication 1, **caractérisé en ce que** de l'azote est injecté au niveau du plateau inférieur de la colonne de réaction (3), pour augmenter la charge en vapeur dans la partie inférieure de la colonne de réaction.

3. Procédé selon la revendication 1 et/ou 2, **caractérisé en ce que** de l'azote est injecté comme agent de rectification au niveau du plateau inférieur de la colonne de réaction (3), en vue de l'élimination supplémentaire de l'eau de réaction.

4. Procédé selon l'une quelconque des revendications 1 à 3, **caractérisé en ce que** l'azote est injecté en vue de la désodorisation au niveau du plateau inférieur de la colonne de réaction (3).

5. Procédé selon l'une quelconque des revendications 1 à 4, **caractérisé en ce que** l'estérification est réalisée à des températures comprises entre 50 et 200°C.

6. Procédé selon l'une quelconque des revendications 1 à 5, **caractérisé en ce qu'**on estérifie les acides (gras) avec des monoalcools en C₁-C₁₀.

7. Procédé selon l'une quelconque des revendications 1 à 6, **caractérisé en ce qu'**on estérifie les acides (gras) avec des dialcanols ou des trialcanols en C₂-C₅.

8. Procédé selon l'une quelconque des revendications 1 à 7, **caractérisé en ce qu'**on utilise comme acides (gras) de préférence des acides gras en C₆-C₂₂.

9. Procédé selon l'une quelconque des revendications 1 à 8, **caractérisé en ce qu'**on estérifie des acides carboxyliques en C₁-C₅ avec des dialcanols ou des trialcanols en C₂-C₃.

10. Procédé selon l'une quelconque des revendications 1 à 9, **caractérisé en ce qu'**on utilise des catalyseurs d'estérification choisis dans le groupe formé par les échangeurs d'anions ou de cations basiques ou acides à base organique ou inorganique ou les argiles réglées à pH acide, les zéolithes ou les terres décolorantes spécialement traitées, ainsi que des catalyseurs à base de métaux de transition.

11. Procédé selon l'une quelconque des revendications 1 à 10, **caractérisé en ce qu'**on utilise, comme catalyseurs, des échangeurs de cations.
